# EUROPEAN PATENT SPECIFICATION

(11) **EP 2 682 066 B1**
(45) Date of publication and mention of the grant of the patent: **06.02.2019**
(21) Application number: 13173886.6
(22) Date of filing: 26.06.2013
(51) Int. Cl.: A61B 18/14, A61B 18/00

(54) **Hand piece with adjustable utility conduit**
Handstück mit einstellbarem Kabelkanal
Pièce à main avec conduit d'utilité ajustable

(30) Priority: 03.07.2012 US 201213541210; 14.03.2013 US 201313831379
(43) Date of publication of application: 08.01.2014
(60) Divisional application: 18191116.5
(73) Proprietor: Megadyne Medical Products, Inc., Draper, UT 84020 (US)
(72) Inventor: Greep, Darcy W., Herriman, UT Utah 84096 (US); Borgmeier, Paul R., Salt Lake City, UT Utah 84124 (US); Frampton, Chad S., American Fork, UT Utah 84003 (US)
(74) Representative: Carpmaels & Ransford LLP

(56) References cited:
- GB-A- 2 452 392
- US-A1- 2010 274 242

## Description

### BACKGROUND

### 1. Technical Field

This invention relates to hand-held instruments. More particularly, the invention relates to hand-held instruments that facilitate the performance of various procedures while reducing the amount of fatigue experienced by users performing the procedures.

### 2. The Relevant Technology

As is known to those skilled in the art, modern surgical techniques typically employ radio frequency (RF) power to cut tissue and coagulate bleeding encountered in performing surgical procedures. For a historical perspective and details of such techniques, reference is made to United States Patent No. 4,936,842, issued to D'Amelio et al., and entitled "Electroprobe Apparatus," the disclosure of which is incorporated by this reference.

As is known to those skilled in the medical arts, electrosurgery is widely used and offers many advantages including the use of a single surgical instrument for both cutting and coagulation. A monopolar electrosurgical generator system has an active electrode, such as in the form of an electrosurgical instrument having a hand piece and a conductive electrode or tip, which is applied by the surgeon to the patient at the surgical site to perform surgery and a return electrode to connect the patient back to the generator.

The electrode or tip of the electrosurgical instrument is small at the point of contact with the patient to produce an RF current with a high current density in order to produce a surgical effect of cutting or coagulating tissue. The return electrode carries the same RF current provided to the electrode or tip of the electrosurgical instrument, thus providing a path back to the electrosurgical generator.

To make the electrical connection for the RF current between the electrosurgical generator and the electrosurgical instrument, a cable having an electrically conductive core extends from the electrosurgical generator to the electrosurgical instrument. The cable may also include a cord with additional conductors. The cord provides a connection for transmitting control signals from the electrosurgical instrument to the electrosurgical generator. The control signals may be used to cause the generator to deliver RF currents to the electrosurgical instrument for different cutting modes such as cut, coagulate, and cut-coagulate blend.

When an electrosurgical instrument is used for cutting or coagulation, smoke is commonly produced. A surgeon or assistant uses a separate smoke evacuation device to remove the smoke from the surgical field. Smoke evacuation devices commonly include a suction wand connected to a vacuum device via tubing. The surgeon or assistant holds the suction wand close to the surgical site and the smoke is drawn into the suction wand and through the tubing. However, using a smoke evacuation device separate from the electrosurgical instrument is not ideal. Using a separate smoke evacuation device requires additional hands and instruments near the surgical site, which can obscure the surgeon's view of the surgical site and reduce the room available around the surgical site for the surgeon to move.

As a result, electrosurgical instrument and smoke evacuation combination devices have been developed. These combination devices often include a hand piece that can receive an electrode or tip for performing electrosurgical procedures. The hand piece is connected to a generator via a power cable to convey RF current to the electrode or tip. Additionally, a smoke evacuation hose is connected between the hand piece and a vacuum to draw smoke away from the surgical site. In some cases, the power cable runs through a portion of the smoke evacuation hose.

The power cables and smoke evacuation hoses have certain flexibility and weight characteristics that limit the ability of the physician during a surgical procedure. For example, the weight/moment-arm effect and drag of the power cable and/or the smoke evacuation hose as well as the connection location(s) of the power cable and/or smoke evacuation hose to the electrosurgical instrument limit the physician's ability to continually hold and use the electrosurgical instrument. The electrode or tip is received within one end of the hand piece (commonly referred to as a pencil) and the power cable and/or smoke evacuation hose typically enter into the opposite end of the hand piece. As the physician manipulates the electrosurgical instrument during a surgical procedure, the weight of the power cable and/or smoke evacuation hose continually pulls on the end of the electrosurgical instrument to which it is attached. More specifically, as the physician moves his or her wrist or adjusts the orientation of the electrosurgical instrument with his or her fingers so as to bring the electrode into contact with the patient's tissue, the weight of the power cable and/or smoke evacuation hose resists the physician's movement. The constant resistance or drag created by the power cable and/or smoke evacuation hose can cause the physician to become fatigued during a surgical procedure that requires extensive and continual use of the electrosurgical instrument.

Additionally, many electrosurgical procedures are performed on very sensitive parts of the body, such as on or around the eyes. When performing such procedures, the physician must control the movements of the electrode with great precision and accuracy. The resistance or drag created by the power cable and/or smoke evacuation hose can make it more difficult for the physician to be as precise and accurate. For instance, when moving the electrosurgical instrument to make a delicate incision, the physician must accurately compensate for the resistance from the power cable and/or smoke evacuation hose. If the physician overcompensates, an incision that is too deep or too long can result. Alternatively, if the physician undercompensates, multiple passes may be required to achieve the desired incision. Furthermore, the fatigue caused by the resistance from the power cable and/or smoke evacuation hose can adversely affect the physician's ability to accurately compensate for the resistance from the power cable and/or smoke evacuation hose.

U.S. Patent No. 8,211,103 discloses an electrosurgical instrument that has an adjustable power cable that can be adjusted to exit the electrosurgical instrument from various positions along the length of the instrument. While the adjustable nature of the power cable may reduce the drag associated with the power cable, a smoke evacuation hose associated with the instrument may still create undesirable resistance. Furthermore, certain characteristics (e.g., size, flexibility) of the power cable disclosed in U.S. Patent No. 8,211,103 may not allow the power cable to provide certain benefits or perform certain functions (e.g., evacuation smoke, act as a stable handle, etc.).

The subject matter claimed herein is not limited to embodiments that solve any disadvantages or that operate only in environments such as those described above. Rather, this background is only provided to illustrate one exemplary technology area where some embodiments described herein may be practiced.

An electrosurgical instrument with adjustable power cable is known from US2010274242A1. Therein is disclosed an electrosurgical instrument for use during an electrosurgical procedure to transmit electrical energy from an electrosurgical generator to tissue of a patient, the electrosurgical instrument comprising an electrical cable coupleable to the electrosurgical generator and configured to transmit the electrical energy from the electrosurgical generator, a hand piece having a proximal end, a distal end, and a central portion disposed therebetween, the central portion having a receptacle configured to receive therein an end of the electrical cable, the hand piece further comprising a channel system that enables a user of the electrosurgical instrument to selectively position at least a portion of the electrical cable within the channel system to thereby select an exit location from the channel system for the electrical cable, wherein the channel system comprises first and second opposing side channels that extend from the receptacle to opposing sides of the hand piece, the first and second opposing side channels being configured to selectively and removably receive at least a portion of the electrical cable therein such that the electrical cable may exit the channel system from the central portion on either side of the hand piece, and a longitudinal channel that extends from the receptacle toward the proximal end, wherein the longitudinal channel comprises a plurality of detents spaced along a length of the longitudinal channel, the plurality of detents being configured to selectively and removably receive at least a portion of the electrical cable therebetween, wherein the plurality of detents defines a plurality of discrete exit locations from which the electrical cable can exit the channel system, wherein the user of the electrosurgical instrument can selectively position the electrical cable within the first side channel, the second side channel, or the longitudinal channel to select the exit location of the electrical cable from the channel system, and a conductive electrode tip configured to be received within the distal end of the hand piece and transmit the electrical energy to the tissue of a patient, wherein the channel system comprises one or more channels that enable the user to selectively adjust an exit location of an evacuation hose that is connected to the hand piece.

### BRIEF SUMMARY

In accordance with the present invention, there is provided a hand-held instrument as defined by claim 1. Further aspects and preferred embodiments are defined in the dependent claims. Aspects, embodiments and examples of the present disclosure which do not fall under the scope of the appended claims do not form part of the invention and are merely provided for illustrative purposes.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only illustrated embodiments of the invention and are therefore not to be considered limiting of its scope. The invention will be described and explained with additional specificity and detail through the use of the accompanying drawings in which:
Figure 1 illustrates an electrosurgical system with an electrosurgical instrument according to one exemplary embodiment of the present invention;
Figure 2 illustrates one manner of holding an electrosurgical instrument;
Figure 3 is a perspective view of an electrosurgical instrument according to an exemplary embodiment of the present invention;
Figure 4 is a bottom perspective view of the electrosurgical instrument of Figure 3 showing a channel system formed in a hand piece thereof;
Figure 5 is a bottom view of the electrosurgical instrument of Figure 3 showing exemplary exit locations for an electrical cable that is connected to the hand piece of the electrosurgical instrument;
Figure 6 is a bottom perspective view of a another exemplary embodiment of an electrosurgical instrument having a channel system;
Figure 7 is a bottom perspective view of the electrosurgical instrument of Figure 6 showing exemplary exit locations for an electrical cable that is connected to the hand piece of the electrosurgical instrument;
Figure 8 is a bottom perspective view of yet another exemplary embodiment of an electrosurgical instrument having a channel system;
Figure 9 illustrates an exemplary electrosurgical instrument being held with the electrical cable exiting the electrosurgical instrument from multiple exemplary exit locations;
Figure 10 is a perspective view of an electrosurgical instrument according to another exemplary embodiment of the present invention;
Figure 11 is a bottom perspective view of the electrosurgical instrument of Figure 10 with a utility conduit removed to show a channel system formed in a hand piece of the electrosurgical instrument;
Figure 12 is a bottom perspective view of the electrosurgical instrument of Figure 10 showing an exemplary exit location for a utility conduit that is connected to the hand piece of the electrosurgical instrument;
Figure 13 is another bottom perspective view of the electrosurgical instrument of Figure 10 showing another exemplary exit location for a utility conduit that is connected to the hand piece of the electrosurgical instrument; and
Figure 14 illustrates the electrosurgical instrument of Figure 10 being held with the utility conduit exiting the electrosurgical instrument from multiple exemplary exit locations.

### DETAILED DESCRIPTION

The present invention relates to hand-held instruments or hand pieces that facilitate the performance of various procedures while reducing the amount of fatigue experienced by users performing the procedures. In some embodiments a hand-held instrument or hand piece is an electrosurgical instrument that holds an electrode tip in one end thereof. The hand piece is connected to a utility conduit. In embodiments that include an electrode tip, the utility conduit may include an electrical cable that is connected to an electrosurgical generator. The utility conduit may also or alternatively include a smoke/fluid evacuation hose that is connected to a vacuum device. Further, the utility conduit may include other tubes, cables, or the like for conveying electrical signals, smoke, fluid, and the like to or from the electrosurgical instrument.

In contrast to most electrosurgical instruments that have an electrical cable and/or a smoke/fluid evacuation hose connected to an end of the hand piece, the electrosurgical instrument of the present invention provides for the utility conduit to be connected to the hand piece at a central portion of the hand piece. The central connection location of the utility conduit to the hand piece reduces the resistance to the movement of the electrosurgical instrument created by the weight/moment-arm effect and drag of the utility conduit. The reduced resistance leads to less fatigue in the physician's hand and arm during electrosurgery. In addition to the central connection location between the hand piece and the utility conduit, the hand piece can be configured to allow the physician to adjust the location on the hand piece at which the utility conduit exits the hand piece. The physician can, therefore, selectively adjust the utility conduit relative to the hand piece in order to customize the electrosurgical instrument to the physician's liking.

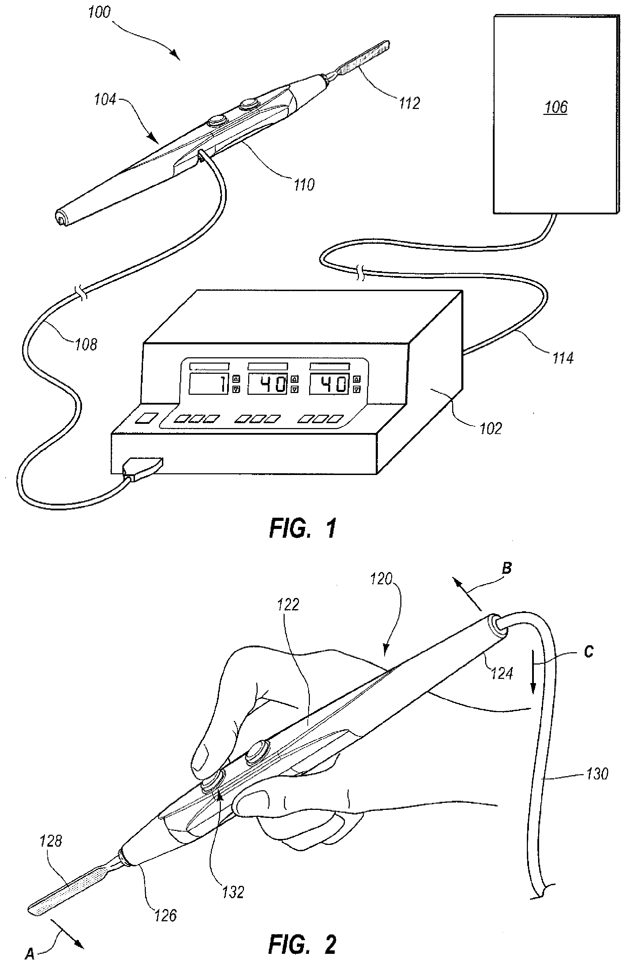

Referring to Figure 1, an exemplary environment is illustrated that provides one operating environment for use of the present invention. In Figure 1, an electrosurgical system 100 is illustrated, which includes a signal generator 102, an electrosurgical instrument 104, and a return electrode 106. Generator 102, in one embodiment, is an RF wave generator that produces RF electrical energy and communicates the RF electrical energy to electrosurgical instrument 104 via cable 108. Cable 108 may be considered an example or component of a utility conduit. Electrosurgical instrument 104 includes a hand piece or pencil 110 and an electrode tip 112. Electrosurgical instrument 104 communicates the RF electrical energy to a patient to cut tissue and/or cauterize blood vessels of the patient's body. Specifically, an electrical discharge is delivered from tip 112 to the patient in order to cause the heating of cellular matter of the patient that is in extremely close contact to tip 112. The heating takes place at an appropriately high temperature to allow electrosurgical instrument 104 to be used to perform electrosurgery. Return electrode 106 and cable 114 provide a return electrical path to wave generator 102 for any excess charge that dissipated into surrounding tissue of the patient's body.

Illustrated in Figure 2 is an electrosurgical instrument 120 commonly used to perform electrosurgical procedures. Electrosurgical instrument 120 includes a hand piece 122 having a proximal end 124 and a distal end 126. An electrode tip 128 is received within distal end 126. A cable 130 is connected to electrosurgical instrument 120 at proximal end 124. Cable 130 communicates electrical energy from an electrosurgical generator, such as generator 102 in Figure 1, to electrosurgical instrument 120. The electrical energy is passed through electrode tip 128 and into a patient's tissue.

Electrosurgical instruments, such as electrosurgical instrument 120, are commonly referred to as electrosurgical pencils or pens because in use they are often held in the same manner as a pencil or pen when writing. Figure 2 illustrates one of the most common manners by which physicians hold electrosurgical instrument 120 during an electrosurgical procedure. As can be seen, hand piece 122 is laid through the crook of the hand and is held in place by the middle finger and thumb. The index finger is placed on top of hand piece 122 to further hold hand piece 122 in place as well as to activate input device 132.

As noted elsewhere herein, the flexibility, weight/moment-arm and drag characteristics of cable 130 and the connection location of cable 130 to hand piece 122 limit the ability of the physician during a surgical procedure. While holding electrosurgical instrument 120 as shown in Figure 2, a physician will perform electrosurgery by activating input device 132 and moving electrode tip 128 into contact with the patient's tissue. To make contact between electrode tip 128 and the patient's tissue, the physician will move his or her wrist or fingers to adjust the position and/or orientation of electrosurgical instrument 120.

For instance, the physician may move his or her wrist so that electrode tip 128 moves in the direction of arrow A toward the patient's tissue. Notably, as the physician moves electrode tip 128 in the direction of arrow A, proximal end 124 moves in the direction of arrow B. The weight of cable 130 constantly pulls proximal end 124 in the direction of arrow C. Thus, the weight of cable 130 resists the movement of proximal end 124 in the direction of arrow B.

The resistance created by the weight of cable 130 is accentuated by the location at which cable 130 is connected to hand piece 122. As is understood, a torque is created by applying a force at a distance from an axis or pivot point. The magnitude of the torque is a result of the magnitude of the applied force and the distance between the axis/pivot point and the location where the force is applied. In the case of electrosurgical instrument 120, the weight of cable 130 is the force that contributes to the generation of the resistive torque. Additionally, the location at which cable 130 attaches to hand piece 122 and how hand piece 122 is held creates the lever arm through which the weight of cable 130 works to create the torque. More specifically, cable 130 enters hand piece 122 at or near proximal end 124. When electrosurgical instrument 120 is held as shown in Figure 2, proximal end 124 is positioned above and away from the crook of the physician's hand, which acts as the pivot point. The weight of cable 130 pulls down on proximal end 124, thereby creating a torque or moment-arm. Because the magnitude of the torque is dependent on the distance between the pivot point and the force, the further apart the connection point between cable 130 and hand piece 122 is away from the crook of the hand, the greater the torque will be. Understandably, the larger the torque is, the greater amount of resistance the physician will experience when manipulating electrosurgical instrument 120.

To overcome the resistance created by the weight of cable 130, the physician must exert additional energy to move electrosurgical instrument 120 into the desired orientation. Continuously working against the resistance created by cable 130 can cause the physician's hand, and/or wrist, and/or arm to become fatigued during an electrosurgical procedure. This fatigue can also lead to a loss of accuracy and precision in the performance of the procedure.

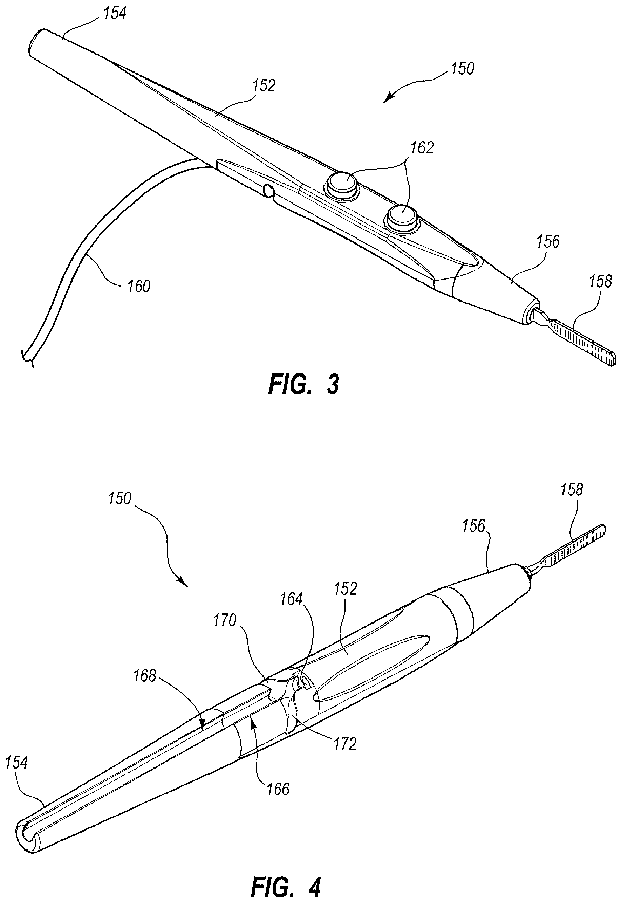

Attention is now directed to Figures 3-8, which illustrate embodiments of electrosurgical instruments which reduce the resistance created by the electrical cable that connects the electrosurgical instruments to an electrosurgical generator. The embodiments shown in Figures 3-8 include a connection point between the electrical cable and the hand piece that is more centrally located between the proximal and distal ends of the hand piece. Additionally, the hand pieces can include one or more grooves or channels into which the cable can be received. The grooves/channels and/or cable can be configured to allow a physician to select the point at which the cable will extend from the hand piece, referred to hereinafter as the "exit location" of the cable. By allowing the physician to select and adjust the exit location, the physician is able to reduce or eliminate the resistance created by the weight of the cable, which can reduce the fatigue experienced during an electrosurgical procedure.

For example, Figures 3-5 illustrate one exemplary embodiment of an electrosurgical instrument 150 according to the present invention. Electrosurgical instrument 150 includes a hand piece 152 having a proximal end 154 and a distal end 156. Distal end 156 is configured to receive an electrode tip 158. Hand piece 152 is connected to cable 160, which delivers electrical energy from a generator, such as generator 102 in Figure 1, to electrode tip 158. Hand piece 152 also includes input devices 162 for controlling the flow of electrical energy to electrode tip 158. As can be seen in Figure 3, cable 160 extends from a central portion of hand piece 152 as opposed to extending from the proximal end of the hand piece as cable 130 does in Figure 2.

Figure 4 illustrates a bottom perspective view of electrosurgical instrument 150. As seen therein, hand piece 152 includes a receptacle 164. An end of cable 160 (not shown in Figure 4) is inserted into receptacle 164 and connected to the internal components of hand piece 152 to provide electrical energy to electrode tip 158. Hand piece 152 also includes a channel system 166 that allows a physician to select the exit location of cable 160. In other words, while cable 160 is connected to hand piece 152 at receptacle 164, a portion of cable 160 can be positioned within channel system 166 so that cable 160 exits or extends from hand piece 152 at any one of a number of locations on hand piece 152, whether adjacent to or distant from receptacle 164.

In the exemplary embodiment illustrated in Figure 4, channel system 166 includes a longitudinal channel 168 and opposing side channels 170 and 172. Each of channels 168, 170, and 172 begins near and extends away from receptacle 164. Longitudinal channel 168 generally extends in a straight line from receptacle 164 to proximal end 154. Opposing side channels 170 and 172 extend from receptacle 164 and out opposing sides of hand piece 152. Channels 168, 170, and 172 are each sized to selectively receive and retain at least a portion of cable 160 therein.

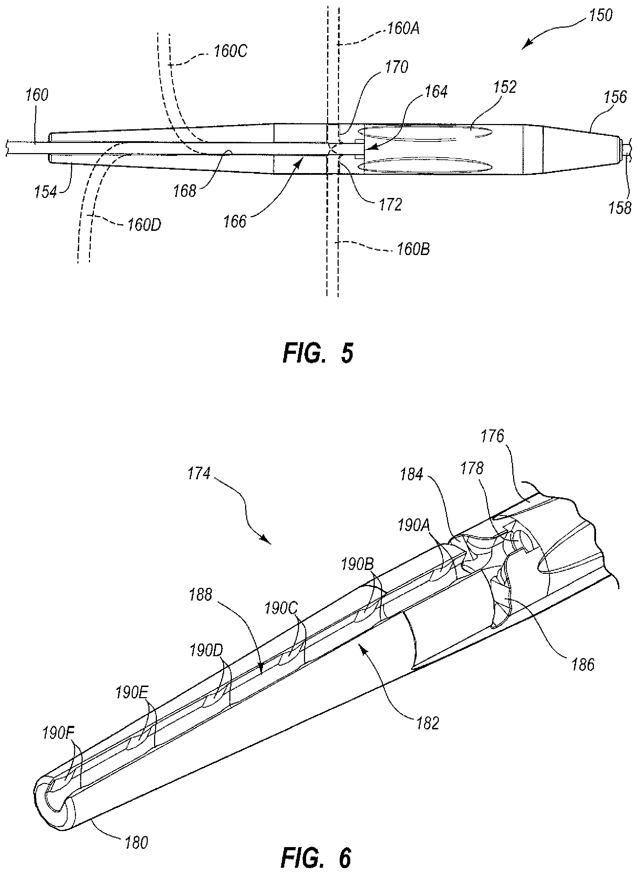

Figure 5 depicts cable 160 exiting or extending away from hand piece 152 at various possible locations. For the sake of clarity, cable 160 is identified in Figure 5 with reference numbers 160, 160A, 160B, 160C, and 160D. While references numbers 160, 160A, 160B, 160C, and 160D identify the same cable 160, the reference letters A, B, C, and D are used to aid in the following description of cable 160 exiting or extending from hand piece 152 at different locations. Similar lettering is also used elsewhere herein with reference to cables 192, 210, and 222 and utility conduit 260.

As shown in Figure 5, cable 160A can extend out of receptacle 164 and be positioned in channel 170 so that cable 160A extends out of the right side of hand piece 152 (when viewed from the top of hand piece 152). As noted above, channel 170 can be sized to snuggly retain cable 160A therein so that cable 160A does not inadvertently come out of channel 170 while electrosurgical instrument 150 is being used. Positioning cable 160A in channel 170 so that cable 160A exits hand piece 152 on the right side may be particularly comfortable for a left-handed physician. When cable 160A is positioned within channel 170, a left-handed physician may hold hand piece 152 so that cable 160A extends out of the right side of hand piece 152 towards the physician's thumb. The physician may allow cable 160A to extend in a downward direction below the thumb. Alternatively, the physician may position cable 160A so that it extends over the top of the thumb before extending in a downward direction.

Similarly, cable 160B can extend out of receptacle 164 and be positioned in channel 172 so that cable 160B extends out of the left side of hand piece 152 (when viewed from the top of hand piece 152). As with channel 170, channel 172 can be sized to snuggly retain cable 160B therein so that cable 160B does not inadvertently come out of channel 172 while electrosurgical instrument 150 is being used. Positioning cable 160B in channel 172 so that cable 160B exits hand piece 152 on the left side may be particularly comfortable for a right-handed physician. A right-handed physician holding hand piece 152 with cable 160B positioned within channel 172 can allow cable 160B to extend below or over the top of the thumb in the same manner as described above with reference to cable 160A and channel 170.

Positioning cable 160A/160B within channel 170/172, as shown in Figure 5, significantly reduces or eliminates the resistance typically created by the electrical cable, as described above with reference to Figure 2. When cable 160A/160B is positioned within channel 170/172, the exit location of cable 160A/160B is relatively close to the crook of the physician's hand. This positioning of the exit location for cable 160A/160B reduces the torque created by the cable. Specifically, because the distance between the applied force (i.e., the weight of the cable) and the pivot point (i.e., the crook of the hand) is relatively small, the torque created by cable 160A/160B is much smaller than the torque created by cable 130 described above with reference to Figure 2. Due to the smaller torque, a physician will experience less resistance and fatigue when using electrosurgical instrument 150 with cable 160A/160B positioned within channel 170/172 as compared to a typical electrosurgical instrument in which the cable is connected at the proximal end.

Some physicians may prefer to have cable 160 exit hand piece 152 at a location other than those provided by channels 170 and 172. In such a case, the physician may use channel 168 to achieve a comfortable exit location for cable 160. To achieve a comfortable exit location, a physician may position a portion of cable 160 within channel 168 so that cable 160 extends from receptacle 164, through channel 168, and exits channel 168 at a desired location between receptacle 164 and proximal end 154.

For instance, with reference to cable 160C of Figure 5, the physician may position about 25.4 mm or 50.8 mm (one or two inches) of cable 160C within channel 168 and then allow the remainder of cable 160C to remain free from hand piece 152. In such a case, cable 160C may exit hand piece 152 so that cable 160C lies in the crook of the hand and then falls in a downward direction near the physician's wrist. Alternatively, with reference to cable 160D of Figure 5, the physician may position between about 50.8 mm and 76.2 mm (two and three inches) of cable 160D within channel 168 and then allow the remainder of cable 160D to remain free from hand piece 152. In yet other situations, the physician may position cable 160 through the entire length of channel 168 so that cable 160 does not exit hand piece 152 until proximal end 154.

While five exit locations have been illustrated in Figure 5, one associated with each of cables 160, 160A, 160B, 160C, and 160D, it will be readily understood that channel system 166 can provide still other exit locations for an electrical cable. Channel system 166 is configured to allow a physician to select any location along the length of channel 168 as an exit location. For instance, a physician with a larger hand may desire cable 160 to exit closer to proximal end 154. This can be accomplished by simply positioning more of cable 160 within channel 168 so that the exit location of cable 160 is closer to proximal end 154. Alternatively, a physician with a smaller hand may desire cable 160 to exit very near receptacle 164. This can be accomplished using channels 170 and 172 or by positioning only a short length of cable 160 within channel 168. Thus, channel system 166 allows a physician to customize hand piece 152 so that hand piece 152 is most comfortable to that physician and reduces the resistance and fatigue caused by cable 160.

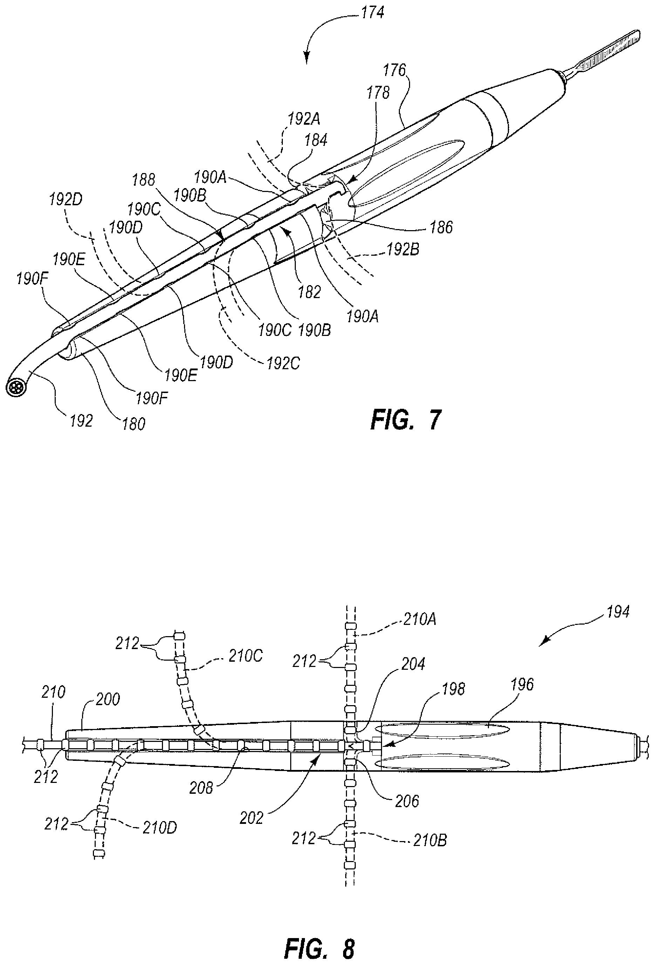

Turning attention now to Figures 6 and 7, there is illustrated an alternative embodiment of a channel system that can be incorporated into an electrosurgical instrument. With specific reference to Figure 6, there is shown an electrosurgical instrument 174 that includes a hand piece 176. Hand piece 176 includes a receptacle 178. An end of cable 192 (Figure 7) is inserted into receptacle 178 and connected to the internal components of hand piece 176 to provide electrical energy to an electrode tip.

Hand piece 176 also includes a channel system 182 similar to channel system 166. Specifically, channel system 182 includes opposing side channels 184 and 186 and a longitudinal channel 188. Opposing side channels 184 and 186 are generally the same as channels 170 and 172 described above. Channel 188 is also similar to channel 168. Channel system 182 allows a physician to select the exit location of cable 192 in much the same manner as described above with reference to channel system 166. More particularly, a physician may select the exit location of cable 192 by positioning cable 192 within one of channels 184, 186, 188. Selection of channels 184 and 186 causes cable 192 to exit the sides of hand piece 176, similar to cable 160 exiting the sides of hand piece 152 through channels 170 and 172. In the case of using channel 188, the physician can also select from a number of predefined exit locations along the length of channel 188. Thus, channel system 182 allows a physician to customize electrosurgical instrument 174 so as to reduce the resistance and fatigue caused by cable 192 and make electrosurgical instrument 174 more comfortable to use.

While channel 188 is similar to channel 168, channel 188 further includes detents 190A, 190B, 190C, 190D, 190E, and 190F, collectively referred to as detents 190. Each of detents 190A, 190B, 190C, 190D, 190E, and 190F includes two opposing ridges positioned within channel 188. Detents 190 are configured to selectively receive and retain cable 192 therebetween. Thus, a physician may position cable 192 within channel 188 and between one or more of detents 190A, 190B, 190C, 190D, 190E, and 190F so as to hold cable 192 while the physician uses electrosurgical instrument 174.

Channel 168, described above, allows a physician to select an exit location anywhere along the length of channel 168. In contrast, detents 190 create a plurality of discrete exit locations for cable 192 along the length of channel 188. In other words, detents 190 provide a physician with the option of selecting an exit location from multiple, predefined exit locations.

As noted, detents 190 are configured to selectively retain cable 192 therebetween. The areas of channel 188 between detents 190 can be configured to allow cable 192 to exit channel 188 when subsequent detents 190A, 190B, 190C, 190D, 190E, and/or 190F are not employed to retain cable 192 within channel 188. For example, as illustrated in Figure 7, a physician may position cable 192C within channel 188 so that cable 192C extends between detents 190A and 190B, but not between detents 190C, 190D, 190E, and 190F. In such a case, cable 192C is then able to exit channel 188 between detents 190B and 190C. Thus, the area between detents 190B and 190C constitutes one of the plurality of discrete exit locations for cable 190. Similarly, the physician may position cable 192D within channel 188 so that cable 192D extends between detents 190A, 190B, 190C, and 190D, but not between detents 190E and 190F. In such a case, cable 192D is then able to exit channel 188 between detents 190D and 190E. Thus, the area between detents 190D and 190E constitutes another one of the plurality of discrete exit locations for cable 190. The areas between detents 190A and 190B, 190C and 190D, and 190E and 190F can similarly constitute discrete exit location for cable 190. While channel 188 is illustrated in Figures 6 and 7 with six detents 190 and five exit locations, it will be understood that a channel system can be formed with fewer or more than six detents 190 and five exit locations.

Channel system 182 therefore provides a physician with the ability to customize electrosurgical instrument 174 in order to reduce the resistance and fatigue caused by cable 192. Additionally, channel system 182 also allows the physician to adjust the exit location of cable 192 to make electrosurgical instrument 174 more comfortable for the physician.

Figure 8 illustrates yet another embodiment of an electrosurgical instrument 194 that reduces the resistance and fatigue caused by the weight and connection location of a cable to the hand piece. In the illustrated embodiment, electrosurgical instrument 194 includes a hand piece 196 having a receptacle 198, a proximal end 200, and a channel system 202. As with receptacles 164 and 178, receptacle 198 is configured to receive an end of cable 210, which provides electrical energy to an electrode tip. Channel system 202 is generally the same as channel system 166. In particular, channel system 202 includes opposing side channels 204 and 206 and a longitudinal channel 208, each of which is sized and configured to selectively receive and retain cable 210 therein.

As with the previous channel systems described herein, channel system 202 allows a physician to select the exit location of cable 210, thereby making electrosurgical instrument 194 more comfortable and reducing the resistance created by the cable. In the present embodiment, however, cable 210 includes a plurality of detents 212 on its outer surface and along its length. Detents 212 provide similar functionality as detents 190 described above. In particular, detents 212 can be sized to snuggly fit within channels 204, 206, and 208 so that cable 210 is retained therein. In addition, detents 212 can be used to select how much of cable 210 is retained within channel system 202. For instance, a physician may position cable 210C within channel 208 so that six detents 212 are held within channel 208, and the remainder of cable 210C is able to hang freely out of channel 208. Alternatively, the physician may position more detents 212 of cable 210 within channel 208, as shown with cable 210D, so that the exit location of cable 210 is closer to proximal end 200. In this manner, the physician may again select from a plurality of predefined, discrete exit location for cable 210, similar to the predefined, discrete exit locations discussed with reference to Figures 6 and 7. In this case, however, the predefined, discrete exit locations are created by detents 212 on cable 210 rather than detents 190 formed in the channel system.

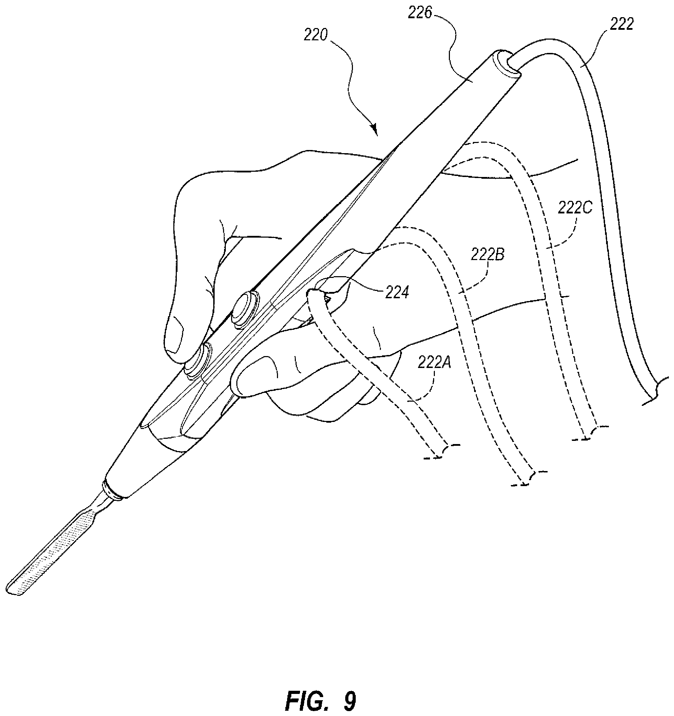

Figure 9 illustrates an electrosurgical instrument 220. Electrosurgical instrument 220 can incorporate any one of the channel systems and/or cables/hoses 222 described herein. Figure 9 illustrates a few examples of where and how a cable or hose 222 exits the electrosurgical instrument 220 and falls relative to a physician's hand. For instance, cable 222A exits electrosurgical instrument 220 through a side channel 224. As can be seen, cable 222A extends out of electrosurgical instrument 220 toward the physician's thumb. Alternatively, the physician can have cable 222B exit electrosurgical instrument 220 on the front side of the physician's palm so that cable 222B is positioned in the palm of the physician's hand. The physician may further select an exit location near the crook of the hand so that cable 222C extends down the crook of the hand towards the wrist. The physician may further select an exit location for cable 222D that is closer to proximal end 226. Still further, the physician may elect to have cable 222 exit electrosurgical instrument 220 at proximal end 226, similar to a standard electrosurgical instrument.

A channel system according to the present invention may also be configured to accommodate multiple cables and/or hoses commonly associated with electrosurgical instruments. For instance, in addition to an electrical cable, many electrosurgical instruments include an evacuation hose to remove smoke or fluid from a surgical site during an electrosurgical procedure. As with the electrical cables, the evacuation hoses are commonly connected to the proximal end of the hand piece, thereby creating resistance to the movement of the electrosurgical instrument. This resistance can, like the resistance from the electrical cables, cause the physician to experience fatigue during an electrosurgical procedure.

To reduce the fatigue caused by the evacuation hoses, a channel system as described herein can be incorporated in the electrosurgical instrument to allow the physician to adjust the exit location of the evacuation hose. For instance, a channel system can be incorporated that allows the evacuation hose to exit the hand piece at a location closer to the physician's hand, thereby reducing the torque created by the weight of the evacuation hose. As described above, the channel system can be configured to allow the physician to select the exit location from anywhere along the length of the hand piece, or from one of a plurality of predefined, discrete exit locations.

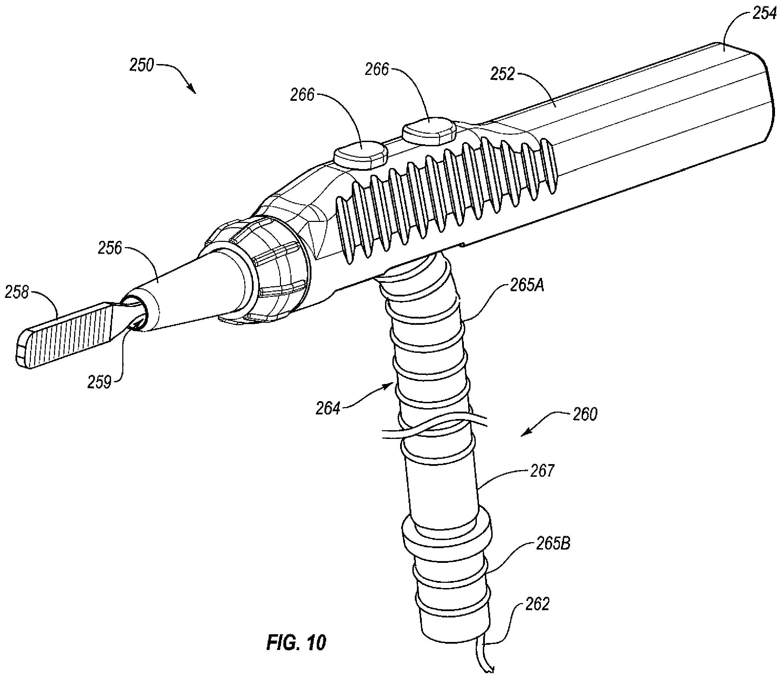

For example, Figures 10-14 illustrate another exemplary embodiment of an electrosurgical instrument 250 according to the present invention. Electrosurgical instrument 250 includes a hand piece 252 having a proximal end 254 and a distal end 256. Distal end 256 is configured as a nozzle that can receive an electrode tip 258 at least partially therein. More specifically, electrode tip 258 is received at least partially within an intake 259 of distal end 256. As can be seen in Figure 10, there is open space between electrode tip 258 and the interior surface of intake 259 to enable smoke and/or fluids to be drawn into intake 259.

Hand piece 252 is connected to a utility conduit 260. As can be seen in Figure 10, utility conduit 260 extends from a central portion of hand piece 252 as opposed to extending from the proximal end of the hand piece as cable 130 does in Figure 2. In the illustrated embodiment, utility conduit 260 includes a cable 262 that delivers electrical energy from a generator, such as generator 102 in Figure 1, to electrode tip 258.

Additionally, utility conduit 260 also includes a smoke/fluid evacuation hose 264 that conveys smoke/fluid away from a surgical site. Smoke/fluid evacuation hose 264 is connected to hand piece 252 so as to be in fluid communication with intake 259. Smoke/fluid evacuation hose 264 may be connected to a vacuum device so as to draw smoke and/or fluid into intake 259, through smoke/fluid evacuation hose 264, and away from a surgical site. Cable 262 or another cable may connect hand piece 252 to the vacuum device so that hand piece 252 may control the vacuum device.

Although utility conduit 260 is described herein as including both a cable 262 and a smoke/fluid evacuation hose 264, such configuration is merely exemplary. In other embodiments a utility conduit may include one or more cables and/or one or more hoses.

In the illustrated embodiment, at least a portion of cable 262 is disposed within smoke/fluid evacuation hose 264. In other embodiments, however, cable 262 may be disposed entirely outside of smoke/fluid evacuation hose 264. In such embodiments, cable 262 and smoke/fluid evacuation hose 264 may optionally be connected to one another along at least a portion of their lengths.

Hand piece 252 also includes input devices 266 for controlling the flow of electrical energy to electrode tip 258 and/or evacuation of smoke and/or fluid from the surgical site.

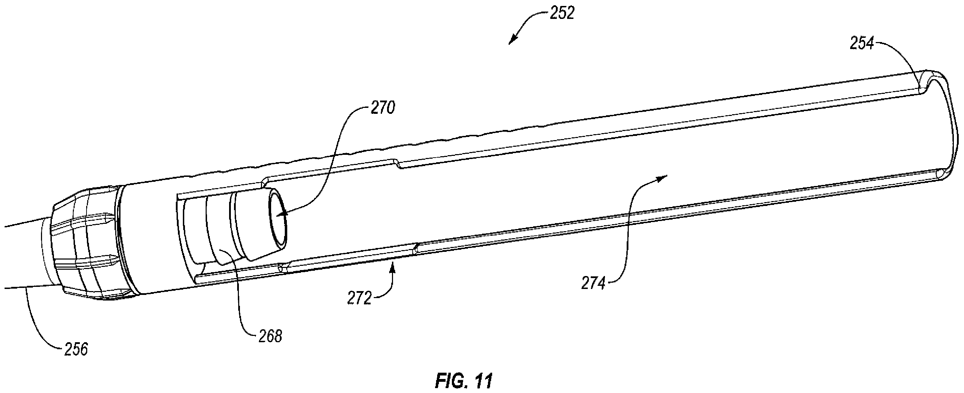

Figure 11 illustrates a bottom perspective view of hand piece 252. As seen therein, hand piece 252 includes a receptacle 268. An end of utility conduit 260 (not shown in Figure 11) may be connected to receptacle 268. For instance, an end of smoke/fluid evacuation hose 264 may be disposed around or received within receptacle 268. As seen in Figure 11, receptacle 268 includes a conduit 270 that is in fluid communication with intake 259. As a result, when smoke/fluid evacuation hose 264 is connected to receptacle 268, smoke/fluid evacuation hose 264 is in fluid communication with intake 259. Accordingly, smoke and/or fluid may be drawn into intake 259, passed through conduit 270, and conveyed away through smoke/fluid evacuation hose 264.

The connection between utility conduit 260 and receptacle 268 may allow for relative movement between hand piece 252 and utility conduit 260. For instance, receptacle 268 may be formed separate from hand piece 252 and later connected thereto. Even more specifically, receptacle 268 may take the form of a swivel that include a first end that may be connected to hand piece 252 and a second end that is connected to utility conduit 260. The connection between the first end of receptacle 268 and hand piece 252, the connection between the first and second ends of receptacle 268 (when formed of two or more pieces), and/or the connection between the second end of receptacle 268 and utility conduit 260 may allow for hand piece 252 to rotate relative to utility conduit 260 or vice versa. For instance, the first end of receptacle 268 may be rotationally connected to hand piece 252 to allow for relative movement between hand piece 252 and utility conduit 260. Similarly, the second end of receptacle 268 may be rotationally connected to utility conduit 260 to allow for similar relative movement between hand piece 252 and utility conduit 260. Likewise, the first and second ends of receptacle 268 may be rotationally connected together to allow for relative movement between hand piece 252 and utility conduit 260. In any case, such relative movement may reduce or eliminate longitudinal rotational torque of hand piece 252 by utility conduit 260.

In addition or as an alternative to hand piece 252 and utility conduit 260 being movably connected together (e.g., a swivel-type receptacle 268), utility conduit 260 may include two or more sections that are connected together in a manner that allows for relative movement between adjacent sections. For instance, as shown in Figures 10 and 12-14, utility conduit 260 may include a first section 265A and a second section 265B that are connected together via a swivel 267. Swivel 267 may include a first half and a second half that are able to rotate relative to one another. First section 265A may be connected to the first half of swivel 267 and second section 265B may be connected to the second half of swivel 267. The ability of the first and second halves of swivel 267 to rotate relative to one another enables first and second sections 265A, 265B of utility conduit 260 to also rotate relative to one another. As a result, hand piece 252 and first section 265A are able to move and rotate relative to second section 265B with less longitudinal rotational torque.

In order to efficiently convey smoke/fluid away from the surgical site, the connections between hand piece 252, receptacle 268, utility conduit 260, first and second sections 265A, 265B, and swivel 267 (and first and second halves thereof), may be substantially airtight and/or watertight.

Although not shown in Figure 11, receptacle 268 also includes electrical connections for connecting cable 262 to hand piece 252. The electrical connection between hand piece 252 and cable 262 allows for controlling the flow of electrical energy to electrode tip 258 and/or removing smoke and/or fluid from the surgical site.

Hand piece 252 also includes a channel system 272 similar to channel systems 166, 182, and 202. Like the other channel systems described herein, channel system 272 allows a physician to select the exit location of utility conduit 260 from hand piece 252. That is, while utility conduit 260 is connected to hand piece 252 at receptacle 268, a portion of utility conduit 260 can be positioned within channel system 272 so that utility conduit 260 exits or extends from hand piece 252 at any one of a number of locations along hand piece 252, whether adjacent to or distant from receptacle 268.

In the exemplary embodiment illustrated in Figure 11, channel system 272 includes a longitudinal channel 274. Longitudinal channel 274 begins near and extends away from receptacle 268 in a generally straight line toward proximal end 254. In other embodiments, longitudinal channel 274 and/or receptacle 268 may be arranged in other configurations. For instance, receptacle 268 may not be aligned with distal end 256 and/or channel 274 may not extend in a generally straight line toward proximal end 254. Rather, channel 274 and/or receptacle 268 may be configured in an offset alignment depending upon the device design and/or desired flow path arrangement.

Longitudinal channel 274 is sized to selectively receive and retain at least a portion of utility conduit 260 therein. That is, longitudinal channel 274 can be sized to snuggly retain utility conduit 260 therein so that utility conduit 260 does not inadvertently come out of longitudinal channel 274 while electrosurgical instrument 250 is being used. As a result of the size and configuration of longitudinal channel 274, hand piece 252 is substantially hollow between receptacle 268 and proximal end 254. For instance, in some embodiments, hand piece 252 is at least 70% hollow. More specifically, in some embodiments, longitudinal channel 274 reduces the overall volume of hand piece 252 by more than 70%. In the illustrated embodiment, for example, hand piece 252 has a width between opposing side surfaces of about 16.5 mm (0.65 inches) and longitudinal channel 274 has a width of about 14 mm (0.55 inches). Accordingly, the ratio between the widths of hand piece 252 and longitudinal channel 274 is about 0.85, resulting in hand piece 252 being about 85% hollow. It will be appreciated, however, that the foregoing percentages and ratios are merely exemplary. By way of non-limiting example, a hand piece may be substantially hollow if the ratio between the width of the hand piece and the width of the longitudinal channel is greater than about 25%, about 40%, about 50%, or about 75%.

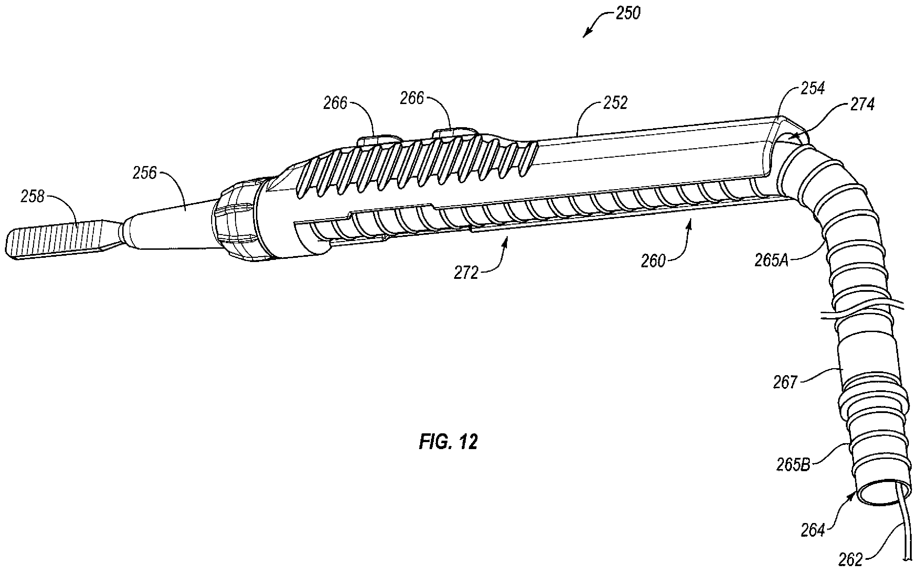

Figure 12 depicts utility conduit 260 exiting or extending away from hand piece 252 at one possible location. As shown in Figure 12, utility conduit 260 can extend away from receptacle 268 (which is generally below input devices 266 in the illustrated embodiment) and be positioned in longitudinal channel 274. In Figure 12, utility conduit 260 is shown disposed in the entire length of longitudinal channel 274 such that utility conduit 260 exits hand piece 252 adjacent proximal end 254.

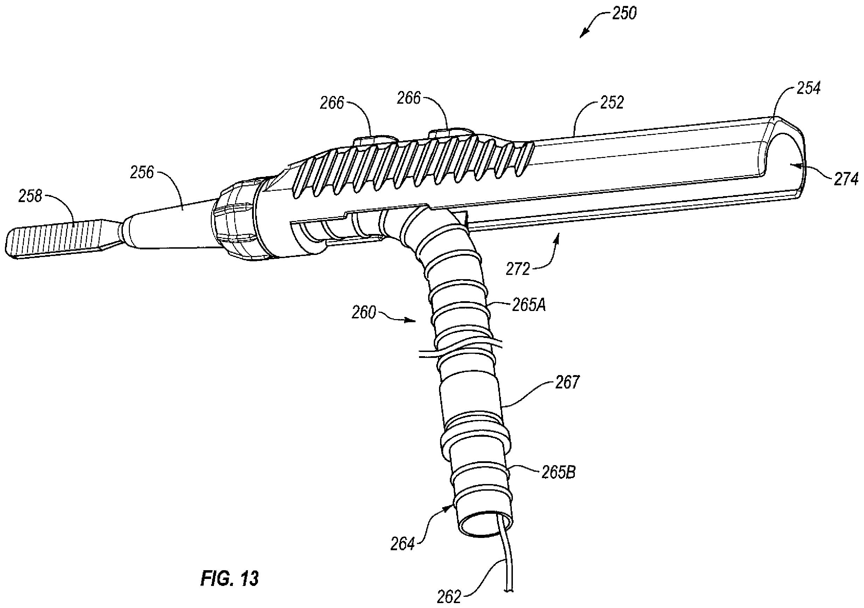

Longitudinal channel 274 allows a physician to select the exit location of utility conduit 260 in much the same manner as described above with reference to longitudinal channels 168, 188, and 208. More particularly, a physician may select the exit location of utility conduit 260 by positioning differing lengths of utility conduit 260 within longitudinal channel 274. For instance, rather that filling the entire length of longitudinal channel 274 with utility conduit 260 as shown in Figure 12, only a portion of longitudinal channel 274 may have utility conduit 260 positioned therein as shown in Figure 13.

Channel system 272 is configured to allow a physician to select substantially any location along the length of longitudinal channel 274 as an exit location. For instance, a physician with a larger hand may desire utility conduit 260 to exit closer to proximal end 254. This can be accomplished by simply positioning more of utility conduit 260 within longitudinal channel 274 so that the exit location of utility conduit 260 is closer to proximal end 254. Alternatively, a physician with a smaller hand may desire utility conduit 260 to exit very near receptacle 268. This can be accomplished by positioning only a short length of utility conduit 260 within longitudinal channel 274. Thus, channel system 272 allows a physician to customize hand piece 252 so that hand piece 252 is most comfortable to that physician and reduces the resistance and fatigue caused by the weight of utility conduit 260.

Notably, by connecting utility conduit 260 adjacent distal end 256, hand piece 252 does not include or act as a smoke/fluid conveying conduit. That is, hand piece 252 does not have to include a channel or other conduit that extends from distal end 256 to proximal end 254 and that is sealed along its entire length. With a typical smoke evacuation device, a first seal is required between the nozzle and a distal end of a conduit in the hand piece and a second seal is required between a proximal end of the conduit in the hand piece and the smoke evacuation hose. Because utility conduit 260 is connected and sealed adjacent to intake 259, a second seal is not required between proximal end 254 and utility conduit 260.

Connecting utility conduit 260 near distal end 256 also provides a flow channel that has a generally uniform diameter from distal end 256 to an associated vacuum device. More specifically, the interior of utility conduit 260 provides a flow channel through which smoke/fluids may be conveyed away from a surgical site. Because utility conduit 260 has a generally uniform inner diameter throughout its length, the flow channel from distal end 256 of hand piece 252 to an associated vacuum device is generally uniform. As will be appreciated by one of ordinary skill in the art, a generally uniform flow channel provides increased flow efficiency of the smoke/fluid being conveyed away. In contrast, common smoke evacuation devices include a flow channel through a hand piece and a smoke evacuation hose that have different diameters, thereby reducing the flow efficiency therethrough.

In the illustrated embodiment, longitudinal channel 274 is shown having a generally straight and smooth interior surface. The generally straight and smooth interior surface of longitudinal channel 274 allows for utility conduit 260 to exit hand piece 252 at substantially any location along the length of longitudinal channel 274. Accordingly, longitudinal channel 274 allows for the exit location of utility conduit 260 to be continuously variable. In other words, the exit location of utility conduit 260 may be selectively adjusted to substantially any location along the length of longitudinal channel 274.

In other embodiments, however, longitudinal channel 274 may optionally be formed with one or more detents, similar to detents 190 shown in Figures 6-7, to facilitate secure holding of utility conduit 260 within longitudinal channel 274 and/or to create one or more predefined, discrete exit locations along the length of longitudinal channel 274. As with detents 190, the exit locations may be defined by the detents in longitudinal channel 274. Channel system 272 may also optionally include one or more side channels similar to side channels 170, 172, 184, 186, 204, and 206.

As can be seen in Figures 10, 12, and 13, at least a portion of utility conduit 260 may be corrugated, convoluted, fluted, or have detents disposed on the outer surface thereof. Like detents 212 described above, the corrugations, convolutions, flutes, or detents on utility conduit 260 may facilitate secure holding of utility conduit 260 within longitudinal channel 274 and/or to create one or more predefined, discrete exit locations along the length of longitudinal channel 274. As with detents 212, the exit locations may be defined by the corrugations, convolutions, flutes, or detents on utility conduit 260 rather than in longitudinal channel 274. It will be understood, however, that utility conduit 260 may not be corrugated, convoluted, fluted, or include detents thereon. Additionally, it is understood that any combination of a utility conduit (corrugated, convoluted, fluted, detented, or smooth) and channel (straight or detented) may be used.

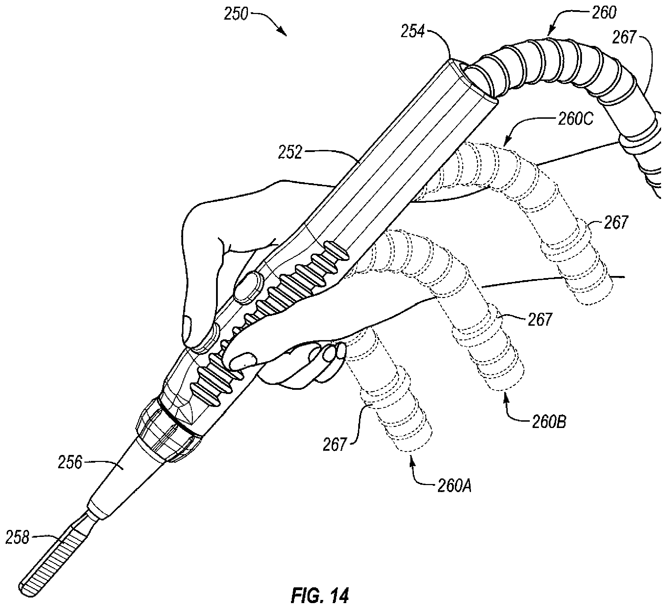

Figure 14 illustrates electrosurgical instrument 250 in use. Specifically, Figure 14 illustrates a few examples of where and how utility conduit 260 may exit hand piece 252 and fall relative to a physician's hand. For instance, utility conduit 260A exits electrosurgical instrument 250 on the front side of the physician's palm so that utility conduit 260A is positioned in the palm of the physician's hand. As a result, the physician may grasp utility conduit 260A by wrapping some or all of his or her fingers around utility conduit 260A. While grasping utility conduit 260A, the physician may also hold hand piece 252 as shown in Figure 14 (e.g., between the thumb and middle finger, with the index finger on top to control the input devices). In this arrangement, utility conduit 260A may act as a handle for electrosurgical instrument 250. Additionally, utility conduit 260A may be formed to provide stability to electrosurgical instrument 250. For instance, utility conduit 260A may be formed of or include a tubing that is stiff enough to maintain the position or orientation of hand piece 252 when utility conduit 260A is used as a handle. More specifically, utility conduit 260A may be stiff enough so that utility conduit 260A maintains hand piece 252 in its current position even when a physician lets go of hand piece 252 and is only holding utility conduit 260A. Furthermore, utility conduit 260A may be sized to comfortably fit within a physician's hand and allow for the physician to securely hold utility conduit 260A.

To provide the above noted stability and grip functionalities, a utility conduit may have an outer diameter of between about 2.5 mm (0.1 inches) and about 76 mm (3 inches). In one example embodiment, a utility conduit has an outer diameter of about 12.7 mm (0.5 inches). A utility conduit may also have some elastic flexibility that contributes to the above-noted functionality. For instance, a utility conduit may be formed to allow for the utility conduit to be angled or bent without collapsing or significantly reducing the inner lumen or flow channel therein. By way of example, the material used to form the utility conduit may allow the utility conduit to have a bend radius of between about 0° and about 180°. In the case of a utility conduit with a bend radius of about 180°, a swivel may be connected between the utility conduit and the hand piece to allow the utility conduit to extend away from the hand piece as shown in the Figures. In other embodiments, all or portions of a utility conduit may be segmented and joined together to provide a moving joint flexibility.

As noted, a utility conduit may be formed from multiple sections. The sections of the utility conduit may have diameters and/or flexibility characteristics that are different from one another. For instance, a first section connected to the hand piece may be relatively stiff to provide the above-noted stability and grip functionalities. In contrast, a second section connected to the first section may be more flexible than the first section.

As noted herein, a utility conduit may be formed by one or more cables and/or one or more hoses. Accordingly, the noted diameters and flexibilities may be a result of multiple hoses, cables, and/or combinations thereof. For instance, two or more hoses may have a combined diameter of between about 2.5 mm (0.1 inches) and about 76 mm (3 inches).

The physician may further select an exit location near the crook of the physician's hand so that utility conduit 260B extends down the crook of the hand towards the wrist. The physician may further select an exit location for utility conduit 260C that is closer to proximal end 254. Still further, the physician may elect to have utility conduit 260 exit electrosurgical instrument 250 at proximal end 254, similar to a standard electrosurgical instrument.

As noted herein, a cable or hose that exits from a proximal end of a hand piece creates resistance, typically in the form of a torque, to the movement of the hand piece. Thus, when a user manipulates the hand piece, either to move the hand piece to a new location or to reorient the hand piece within the same general location, the cable or hose resists the movement or reorientation of the hand piece. Accordingly, allowing the exit location of utility conduit 260 to be adjusted along the length of hand piece 252 reduces the amount of resistance typically created by the cable or hose.

In addition to reducing the overall resistance typically created by a cable or hose, moving the exit location of utility conduit 260 closer to the distal end of hand piece 252 also reduces the change is resistance experienced when moving or reorienting hand piece 252. As a hand piece 252 is moved or reoriented, the resistance created by a cable or hose changes. While the change in resistance may be due at least in part to the direction of movement or reorientation and/or the speed of the movement, the change in resistance is primarily due to the exit location of the cable or hose. As discussed herein, the increased distance between the exit location and the pivot point of the hand piece creates a larger torque. As a result, when the exit location of the cable or hose is at or near the proximal end of the hand piece, the change in resistance during movement or reorientation of the hand piece is greater than the change in resistance created when the exit location is closer to the distal end of the hand piece.

With reference to Figure 14, for instance, when utility conduit 260 extends out of proximal end 254 of hand piece 252, utility conduit 260 creates resistance to the movement of hand piece 252. Additionally, as hand piece 252 is moved or reoriented, the resistance created by utility conduit 260 changes. When utility conduit 260 exits hand piece 252 from a location closer to the distal end of hand piece 252, the resistance created by utility conduit 260 is reduced. Additionally, the change in resistance created by utility conduit 260 when hand piece 252 is moved or reoriented is also reduced as the exit location of utility conduit 260 moves toward the distal end of hand piece 252. When utility conduit 260 exits near the distal end of hand piece 252 (e.g., as illustrated by utility conduit 260A), the resistance and change in resistance created by utility conduit 260 falls dramatically. In such arrangements, the resistance and change in resistance may drop to near zero or at least negligible levels.

The following tables demonstrate that the amount of torque resulting from a distally located exit location is significantly lower than when the exit location is disposed at or near the proximal end of a hand piece. The torque resulting from the cables and/or hoses connected to numerous hand pieces were measured. Specifically, the torques associated with eleven different devices were measured at various heights and at various orientations. Devices 1-4 were standard electrosurgical instruments that include power cables extending from the proximal ends of the hand pieces. Devices 5-10 were electrosurgical instruments that include both power cables and smoke evacuation hoses extending from the proximal ends of the hand pieces. The torque associated with electrosurgical instrument 250 was also measured with utility conduit 260 extending from two different exit locations. The first exit location was at proximal end 254 as shown in Figure 12. The second exit location was below user inputs 266 as shown in Figure 13.

Table 1 includes the torques associated with the eleven devices when the hand pieces were in a level orientation (i.e., the proximal and distal ends of the hand pieces were at substantially the same height). In contrast, Table 2 includes the torques associated with the eleven devices when the hand pieces were held at a 45° angle with the distal end of the hand piece being disposed lower than the proximal end. In addition to measuring the torques when the devices were at different orientations, the torques were also measured when the hand pieces were held at different heights (i.e., 2.5 ft, 3 ft, 3.5 ft, and 4 ft).

Tables 1 and 2 also include other basic information regarding each of the evaluated devices. This information includes the lengths of the hand pieces, the masses of the hand piece and associated cables/hoses, and the distances between the pivot points of the hand pieces and the ends of the hand pieces. To provide consistency throughout the samples, the pivot point for each hand piece was determined to be at the user input button positioned closest to the proximal end of the hand piece.

**TABLE 1**

| | **Height above floor** | | | | **Center of Proximal Input Button to Tip (in.)** | **Center of Proximal Input Button to exit location (in.)** | **Total (in.)** | **Mass of hand piece, cord, & tubing (g)** |
|---|---|---|---|---|---|---|---|---|
| Electrosurgical Devices | 2.5 ft | 3.0 ft | 3.5 ft | 4.0 ft | | | | |
| | **Torque (oz. in.)** | | | | | | | |
| Device 1 | 1.25 | 1.5 | 1.75 | 2 | 3.82 | 3.82 | 7.64 | 73.24 |
| Device 2 | 2 | 2.5 | 3 | 3.5 | 3.85 | 3.8 | 7.65 | 104.19 |
| Device 3 | 0.65 | 0.9 | 1.2 | 1.5 | 4.1 | 3.82 | 7.92 | 62.69 |
| Device 4 | 0.65 | 0.8 | 1 | 1.25 | 4.2 | 3.3 | 7.50 | 66.3 |
| | | | | | | | | |
| Smoke Evac Devices | | | | | | | | |
| Electrosurgical Instrument 250 (utility conduit extending from proximal end) | 5 | 5.75 | 6.75 | 7 | 3.83 | 3.83 | 7.66 | 146.81 |
| Electrosurgical Instrument 250 (utility conduit exiting near input button) | 0 | 0 | 0 | 0 | 3.82 | 0.15 | 3.97 | 146.81 |
| Device 5 | 4.5 | 5.5 | 6.5 | 7 | 4.2 | 4 | 8.20 | 216.27 |
| Device 6 | 1.75 | 3 | 4.5 | 7.5 | 3.97 | 3.96 | 7.93 | 268.73 |
| Device 7 | 4.5 | 5.5 | 6.5 | 7.5 | 3.33 | 3.5 | 6.83 | 180.87 |
| Device 8 | 3.75 | 5.75 | 6 | 6.75 | 4.43 | 3.7 | 8.13 | 141.64 |
| Device 9 | 3.25 | 4.5 | 5.25 | 6.25 | 4.24 | 4.24 | 8.48 | 157.88 |
| Device 10 | 1.5 | 1.75 | 2 | 2.25 | 4.12 | 2.57 | 6.69 | 128.73 |

**TABLE 2**

| | **Height above floor** | | | | **Center of Proximal Input Button to Tip (in.)** | **Center of Proximal Input Button to exit location (in.)** | **Total (in.)** | **Mass of hand piece, cord, & tubing (g)** |
|---|---|---|---|---|---|---|---|---|
| Electrosurgical Devices | 2.5 ft | 3.0 ft | 3.5 ft | 4.0 ft | | | | |
| | **Torque (oz. in.)** | | | | | | | |
| Device 1 | 1.5 | 1.75 | 2 | 2.3 | 3.82 | 3.82 | 7.64 | 73.24 |
| Device 2 | 2.25 | 2.75 | 3.25 | 3.5 | 3.85 | 3.8 | 7.65 | 104.19 |
| Device 3 | 0.7 | 1.25 | 1.5 | 1.75 | 4.1 | 3.82 | 7.92 | 62.69 |
| Device 4 | 1 | 1.25 | 1.5 | 1.75 | 4.2 | 3.3 | 7.50 | 66.3 |
| | | | | | | | | |
| Smoke Evac Devices | | | | | | | | |
| Electrosurgical Instrument 250 (utility conduit extending from proximal end) | 5 | 5.75 | 6.75 | 7 | 3.83 | 3.83 | 7.66 | 146.81 |
| Electrosurgical Instrument 250 (utility conduit exiting near input button) | 0 | 0 | 0 | 0 | 3.82 | 0.15 | 3.97 | 146.81 |
| Device 5 | 4.5 | 5.5 | 6.5 | 7 | 4.2 | 4 | 8.20 | 216.27 |
| Device 6 | 1.75 | 3 | 4.5 | 7.5 | 3.97 | 3.96 | 7.93 | 268.73 |
| Device 7 | 4.5 | 5.5 | 6.5 | 7.5 | 3.33 | 3.5 | 6.83 | 180.87 |
| Device 8 | 3.75 | 5.75 | 6 | 6.75 | 4.43 | 3.7 | 8.13 | 141.64 |
| Device 9 | 3.25 | 4.5 | 5.25 | 6.25 | 4.24 | 4.24 | 8.48 | 157.88 |
| Device 10 | 1.5 | 1.75 | 2 | 2.25 | 4.12 | 2.57 | 6.69 | 128.73 |

As can be seen from the data in Tables 1 and 2, the power cables for the standard electrosurgical devices create torques ranging from 0.45 N-cm (0.65 oz. in.) to 2.47 N-cm (3.5 oz. in.) in the horizontal orientation and from 0.494 N-cm (0.7 oz. in.) to 2.47 N-cm (3.5 oz. in.) in the angled orientation. Similarly, the power cables and hoses for Devices 5-10 create torques ranging from 1.05 N-cm (1.5 oz. in.) to 5.29 N-cm (7.5 oz. in.) in both the horizontal and angled orientations. It is observed that the torque for each device generally increases as the height of the hand piece increases. This is understandable since the length, and thus the weight, of the suspended portion of the power cable and/or evacuation hose increases as the height of the hand piece increases.

With regard to electrosurgical instrument 250, it is noted that the torque from utility conduit 260 is significantly higher when utility conduit exits hand piece 252 at proximal end 254 than when utility conduit 260 exits hand piece 252 near user inputs 266. Specifically, when utility conduit exits hand piece 252 at proximal end 254, utility conduit 260 creates torques ranging from 3.53 N-cm (5 oz. in.) to 4.94 N-cm (7 oz. in.), depending on the height of hand piece 252. In contrast, when utility conduit 260 exits hand piece 252 near user inputs 266, utility conduit 260 creates no torque, or negligible levels of torque. Thus, by allowing the exit location of utility conduit 260 to be adjusted along the length of hand piece 252, a user can customize electrosurgical instrument 250 to provide torques ranging anywhere from about 0 N-cm (0 oz. in.) up to about 4.94 N-cm (7 oz. in.) or more.

It is understood that the features of the above described embodiments are not exclusive to one another. Rather, one of ordinary skill in the art will recognize that the described features can be combined and/or modified as may be needed or desired. For example, the channels of the various channel systems can have generally smooth interior surfaces or they can be formed with detents, either of which can receive a cable/hose/conduit with a generally smooth outer surface. Alternatively, the channels, whether with smooth surfaces or detents, can receive a cable/hose/conduit that has detents of its own formed thereon.

Furthermore, while some of the channel systems have been shown and described as having two opposing side channels and a single longitudinal channel, it will be understood that a channel system according to the present invention can be formed with a single side channel, a single longitudinal channel, multiple side channels, multiple longitudinal channels, or a combination thereof. For instance, a channel system may include one or more side channels along the length of the hand piece, each of which exits to the same side of the hand piece. A channel system may also have multiple side channels on each side of the hand piece. Furthermore, a channel system may have multiple channels, some sized for an electrical cable and some sized for an evacuation hose. Still further, a channel system may have one or more channels that are configured to receive and selectively retain an electrical cable and an evacuation hose in the same channel at the same time.

While the embodiments disclosed herein have been directed to electrosurgical instruments with adjustable utility conduits, the present invention is not intended to be limited only to electrosurgical instruments. Rather, the present invention is broadly directed to any hand-held instrument that has an adjustable utility conduit as described herein. More specifically, the present invention includes any hand-held instrument that has a channel system that allows for the selective adjustment of an exit location of a utility conduit from the hand-held instrument between proximal and distal ends of the hand-held instrument. Examples of such hand-held instruments may include, but are not limited to, dental instruments (e.g., drills, polishing tools, scalers, compressed air tools, suction tools, irrigation tools, carries detection tools, water flossing tool (e.g., waterpik)), soldering tools (e.g., heated tools, smoke collection tools, de-soldering tools), high speed grinding and polishing tools (e.g., Dremel tools, carving tools, manicure tools, dental lab grinders/polishers), laser treatment instruments, laser surgical instruments, light probes, suction handles (e.g., Yankauer), blasting tools (e.g., sandblast, gritblast), shockwave therapy tools, ultrasonic therapy tools, ultrasonic probe tools, ultrasonic surgical tools, adhesive application instruments, glue guns, pneumatic pipettes, welding tools, RF wrinkle therapy hand pieces, phaco hand pieces, shears, shaver, or razor hand pieces, micro drill hand pieces, vacuum hand pieces, small parts handling hand pieces, tattoo needle handles, small torch hand pieces, electrology hand pieces, low speed grinding, polishing and carving tools, permanent makeup hand pieces, electrical probe hand pieces, ferromagnetic surgical hand pieces, surgical suction instruments (e.g., liposuction cannulas), surgical suction cannulas, microdermabrasion hand pieces, fiberoptic camera handles, microcamera hand pieces, pH probe hand pieces, fiberoptic and LED light source hand pieces, hydrosurgery hand pieces, orthopedic shaver, cutter, burr hand pieces, wood burning tools, electric screwdrivers, electronic pad styluses, and the like.

Furthermore, the present invention is not limited to hand-held instruments that allow for the adjustment of an exit location of a utility conduit therefrom. Rather, the present invention also encompasses hand-held instruments that have a utility conduit connected thereto and that extend therefrom at a location other than at the proximal end of the hand-held instrument. For instance, the exit location of the utility conduit may not be adjustable along the length of the hand-held instrument. Nevertheless, the exit location of the utility conduit may be positioned at a location along the length of the hand-held instrument that is away from the distal end of the hand-held instrument.

Even more specifically, the hand-held instrument may have a central portion disposed approximately midway between the proximal and distal ends of the instrument. The exit location of the utility conduit may be positioned at about the central portion of the hand-held instrument or between the central portion and the distal end of the hand-held instrument. The hand-held instrument may also have a three-quarter portion disposed approximately midway between the central portion and the proximal end. In other words, approximately one quarter of the length of the hand-held instrument is disposed between the proximal end and the three-quarter portion, while three quarters of the length of the hand-held instrument are disposed between the three-quarter portion and the distal end. The exit location of the utility conduit may be positioned at about the three-quarter portion of the hand-held instrument or between the three-quarter portion and the distal end of the hand-held instrument.

By way of example, the exit location may be positioned near a central portion of the hand-held instrument or between the central portion and the distal end of the hand-held instrument, similar to cables 160A and 160B in Figure 5, cables 210A and 210B in Figure 8, cable 222A in Figure 9, utility conduit 260 in Figures 10 and 13, or utility conduit 260A in Figure 14. The exit location may also be positioned between the central portion and the proximal end of the hand-held instrument, but away from the proximal end of the hand-held instrument. Positioning the exit location, whether fixed or adjustable, away from the proximal end of the hand-held instrument reduces the resistance from the utility conduit and thereby reduces the fatigue experienced by user of the hand-held instrument.

The present invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. A hand-held instrument (250) comprising:
a hand piece (252) configured to be held by a user, the hand piece having a proximal end (254) and a distal end (256) and comprising:
a receptacle (268) disposed between the proximal end (254) and the distal end (256);
a channel system (272) that extends along a length of the hand piece (252) between the proximal end (254) and the distal end (256);
a utility conduit (260) connected to the receptacle (268); and
the utility conduit (260) comprises a smoke/fluid evacuation hose (264) configured to convey smoke/fluid to or from the hand piece and one or more electrical cables, the utility conduit being configured to be selectively received and retained within at least a portion of the channel system (272), wherein the utility conduit (260) and the channel system (272) cooperate to enable an exit location of the utility conduit from the channel system to be selectively adjusted such that the exit location may be selectively moved along a length of the hand piece (252) between the receptacle (268) and the proximal end (254); wherein, when the utility conduit (260) exits the channel system (272) from at least a distal-most exit location, the utility conduit (260) is configured to be held as a handle in a user's hand to provide additional stability to the hand piece, wherein the utility conduit (260) comprises a first section and a second section, wherein the first section is connected to the hand piece and is stiff enough to generally maintain the position or orientation of the hand piece (252) when the utility conduit (260) is used as a handle, and the second section is connected to the first section, and is more flexible than the first section.

2. The hand-held instrument (250) of claim 1, wherein the hand-held instrument is selected from the group consisting of a medical instrument, a dental instrument, a soldering tool, a wood burning tool, a drill, and an adhesive applicator.

3. The hand-held instrument (250) of claims 1 or 2, wherein the receptacle (268) comprises a swivel that enables relative rotation between the hand piece (252) and the utility conduit (260).

4. The hand-held instrument of any of claims 1-3, comprising a first exit location wherein the utility conduit exits the hand piece at the proximal end of the hand piece and the utility conduit and the channel system (272) cooperate to create a torque on the hand piece in a range of between about 3.53 N-cm (5.0 oz. in.) and about 4.94 N-cm (7.0 oz. in).

5. The hand-held instrument of any of claims 1-4, comprising a second exit location wherein the utility conduit exits the hand piece adjacent to the receptacle and the utility conduit and the channel system (272) cooperate to create a torque on the hand piece of about 0 N-cm (0 oz. in).

6. The hand-held instrument (250) of any of claims 1-5, wherein the receptacle (268) is disposed generally below one or more user inputs (266) on the hand piece (252).

7. The hand-held instrument (250) of any of claims 1-6, wherein the channel system (272) comprises one or more discrete exit locations between the receptacle (268) and the proximal end (254).

8. The hand-held instrument (250) of any of claims 1-7, wherein the utility conduit (260) comprises multiple smoke/fluid evacuation hoses (264).

9. The hand-held instrument of any of claims 1-8, wherein the utility conduit (260) has an outer diameter of about 12.7 mm (0.5 inches).

10. The hand-held instrument (250) of any of claims 1-9, wherein the utility conduit (260) comprises a first section (265A) and a second section (265B), wherein the first section and second section are configured for relative rotational movement.

11. The hand-held instrument (250) of claim 10, further comprising a swivel (267) connected between the first section (265A) and the second section (265B) to facilitate relative rotational movement.

12. The hand-held instrument (250) of any of claims 1-11, wherein the hand piece (252) is substantially hollow between the receptacle (268) and the proximal end (254).

13. The hand-held instrument (250) of any of claims 1-12, wherein the channel system (272) comprises a longitudinal channel (274) extending from the receptacle to the proximal end (254) of the hand piece (252).

14. The hand-held instrument (250) of any of claims 1-13, wherein the utility conduit (260) defines an interior flow channel having a generally uniform diameter throughout the length of the utility conduit.

## Patentansprüche

1. In der Hand zu haltendes Instrument (250), das Folgendes umfasst:
ein Handstück (252), das dafür konfiguriert ist, durch einen Benutzer gehalten zu werden, wobei das Handstück ein proximales Ende (254) und ein distales Ende (256) aufweist und Folgendes umfasst:
eine Aufnahme (268), die zwischen dem proximalen Ende (254) und dem distalen Ende (256) angeordnet ist,
ein Kanalsystem (272), das sich entlang einer Länge des Handstücks (252) zwischen dem proximalen Ende (254) und dem distalen Ende (256) erstreckt,
eine Versorgungsleitung (260), die mit der Aufnahme (268) verbunden ist und wobei die Versorgungsleitung (260) einen Rauch-/Fluid-Ableitungsschlauch (264), der dafür konfiguriert ist, Rauch/Fluid zu oder von dem Handstück zu befördern, und ein oder mehrere elektrische Kabel umfasst, wobei die Versorgungsleitung dafür konfiguriert ist, selektiv innerhalb wenigstens eines Abschnitts des Kanalsystems (272) aufgenommen und festgehalten zu werden, wobei die Versorgungsleitung (260) und das Kanalsystem (272) zusammenwirken, um zu ermöglichen, dass eine Austrittsposition der Versorgungsleitung aus dem Kanalsystem derart selektiv eingestellt werden kann, dass die Austrittsposition selektiv entlang einer Länge des Handstücks (252) zwischen der Aufnahme (268) und dem proximalen Ende (254) bewegt werden kann, wobei, wenn die Versorgungsleitung (260) aus dem Kanalsystem (272) aus einer am weitesten distalen Position austritt, die Versorgungsleitung (260) dafür konfiguriert ist, als ein Griff in der Hand eines Benutzers gehalten zu werden, um zusätzliche Stabilität für das Handstück bereitzustellen, wobei die Versorgungsleitung (260) eine erste Sektion und eine zweite Sektion umfasst, wobei die erste Sektion mit dem Handstück verbunden ist und steif genug ist, um im Allgemeinen die Position oder Ausrichtung des Handstücks (252) aufrechtzuerhalten, wenn die Versorgungsleitung (260) als ein Griff verwendet wird, und die zweite Sektion mit der ersten Sektion verbunden ist und flexibler ist als die erste Sektion.

2. In der Hand zu haltendes Instrument (250) nach Anspruch 1, wobei das in der Hand zu haltende Instrument ausgewählt ist aus der Gruppe, die aus einem medizinischen Instrument, einem zahnärztlichen Instrument, einem Lötwerkzeug, einen Holzbrennwerkzeug, einem Bohrer und einem Klebstoffapplikator besteht.

3. In der Hand zu haltendes Instrument (250) nach Anspruch 1 oder 2, wobei die Aufnahme (268) ein Drehgelenk umfasst, das eine relative Drehung zwischen dem Handstück (252) und der Versorgungsleitung (260) ermöglicht.

4. In der Hand zu haltendes Instrument nach einem der Ansprüche 1 bis 3, die eine erste Austrittsposition umfasst, wobei die Versorgungsleitung am proximalen Ende des Handstücks aus dem Handstück austritt und die Versorgungsleitung und das Kanalsystem (272) zusammenwirken, um an dem Handstück ein Drehmoment in einem Bereich von zwischen etwa 3,53 Ncm (5,0 Unze-Zoll) und etwa 4,94 zu erzeugen Ncm (5,0 Unze-Zoll) zu erzeugen.

5. In der Hand zu haltendes Instrument nach einem der Ansprüche 1 bis 4, die eine zweite Austrittsposition umfasst, wobei die Versorgungsleitung angrenzend an die Fassung aus dem Handstück austritt und die Versorgungsleitung und das Kanalsystem (272) zusammenwirken, um an dem Handstück ein Drehmoment in einem Bereich von zwischen etwa 0 Ncm (0 Unze-Zoll) zu erzeugen.

6. In der Hand zu haltendes Instrument (250) nach einem der Ansprüche 1 bis 5, wobei die Aufnahme (268) im Allgemeinen unterhalb einer oder mehrerer Benutzereingaben (266) an dem Handstück (252) angeordnet ist.

7. In der Hand zu haltendes Instrument (250) nach einem der Ansprüche 1 bis 6, wobei das Kanalsystem (272) eine oder mehrere diskrete Austrittspositionen zwischen der Aufnahme (268) und dem proximalen Ende (254) umfasst.

8. In der Hand zu haltendes Instrument (250) nach einem der Ansprüche 1 bis 7, wobei die Versorgungsleitung (260) mehrere Rauch-/Fluid-Ableitungsschläuche (264) umfasst.

9. In der Hand zu haltendes Instrument nach einem der Ansprüche 1 bis 8, wobei die Versorgungsleitung (260) einen Außendurchmesser von etwa 12,7 mm (0,5 Zoll) aufweist.

10. In der Hand zu haltendes Instrument (250) nach einem der Ansprüche 1 bis 9, wobei die Versorgungsleitung (260) eine erste Sektion (265A) und eine zweite Sektion (265B) umfasst, wobei die erste Sektion und die zweite Sektion für eine relative Drehbewegung konfiguriert sind.

11. In der Hand zu haltendes Instrument (250) nach Anspruch 10, das ferner ein Drehgelenk (267) umfasst, das zwischen der ersten Sektion (265A) und der zweiten Sektion (265B) angeschlossen ist, um eine relative Drehbewegung zu erleichtern.

12. In der Hand zu haltendes Instrument (250) nach einem der Ansprüche 1 bis 11, wobei das Handstück (252) zwischen der Aufnahme (268) und dem proximalen Ende (254) im Wesentlichen hohl ist.

13. In der Hand zu haltendes Instrument (250) nach einem der Ansprüche 1 bis 12, wobei das Kanalsystem (272) einen Längskanal (274) umfasst, der sich von der Aufnahme zu dem proximalen Ende (254) des Handstücks (252) erstreckt.

14. In der Hand zu haltendes Instrument (250) nach einem der Ansprüche 1 bis 13, wobei die Versorgungsleitung (260) einen inneren Durchflusskanal definiert, der über die gesamte Länge der Versorgungsleitung einen im Wesentlichen gleichmäßigen Durchmesser aufweist.

## Revendications

1. Instrument à main (250) comprenant:
une pièce à main (252) configurée pour être tenue par un utilisateur, la pièce à main ayant une extrémité proximale (254) et une extrémité distale (256) et comprenant:
un réceptacle (268) disposé entre l'extrémité proximale (254) et l'extrémité distale (256);
un système de canal (272) qui s'étend sur une longueur de la pièce à main (252) entre l'extrémité proximale (254) et l'extrémité distale (256);
un conduit d'utilité (260) relié au réceptacle (268); et le conduit d'utilité (260) comprend un tuyau d'évacuation de fumée/fluide (264) configuré pour acheminer de la fumée/fluide vers ou depuis la pièce à main et un ou plusieurs câbles électriques, le conduit d'utilité étant configuré pour être reçu et retenu sélectivement dans au moins une partie du système de canal (272), le conduit d'utilité (260) et le système de canal (272) coopérant pour permettre à un emplacement de sortie du conduit d'utilité du système de canal d'être ajusté sélectivement de sorte que l'emplacement de sortie peut être déplacé sélectivement le long d'une longueur de la pièce à main (252) entre le réceptacle (268) et l'extrémité proximale (254); lorsque le conduit d'utilité (260) sort du système de canal (272) depuis au moins un emplacement de sortie le plus distal, le conduit d'utilité (260) étant configuré pour être tenu comme une poignée dans la main d'un utilisateur pour fournir une stabilité supplémentaire à la pièce à main, le conduit d'utilité (260) comprenant une première section et une seconde section, la première section étant reliée à la pièce à main et étant suffisamment rigide pour maintenir généralement la position ou l'orientation de la pièce à main (252) lorsque le conduit d'utilité (260) est utilisé comme poignée, et la seconde section est reliée à la première section et est plus flexible que la première section.

2. Instrument à main (250) selon la revendication 1, dans lequel l'instrument à main est choisi dans le groupe constitué par un instrument médical, un instrument dentaire, un outil de brasage, un outil de chauffage au bois, une perceuse et un applicateur de colle.

3. Instrument à main (250) selon les revendications 1 ou 2, **caractérisé en ce que** le réceptacle (268) comporte un pivot qui permet une rotation relative entre la pièce à main (252) et le conduit d'utilité (260).

4. Instrument à main selon l'une quelconque des revendications 1 à 3, comprenant un premier emplacement de sortie dans lequel le conduit d'utilité sort de la pièce à main à l'extrémité proximale de la pièce à main et le conduit d'utilité et le système de canal (272) coopèrent pour créer un couple sur la pièce à main dans une plage comprise entre environ 3,53 N-cm (5,0 oz po) et environ 4,94 N-cm (7,0 oz po).

5. Instrument à main selon l'une quelconque des revendications 1 à 4, comprenant un second emplacement de sortie dans lequel le conduit d'utilité sort de la pièce à main adjacente au réceptacle et le conduit d'utilité et le système de canal (272) coopèrent pour créer un couple sur la pièce à main d'environ 0 N-cm (0 oz po).

6. Instrument à main (250) selon l'une quelconque des revendications 1 à 5, dans lequel le réceptacle (268) est disposé généralement au-dessous d'une ou plusieurs entrées utilisateur (266) sur la pièce à main (252).

7. Instrument à main (250) selon l'une quelconque des revendications 1 à 6, dans lequel le système de canal (272) comprend un ou plusieurs emplacements de sortie discrets entre le réceptacle (268) et l'extrémité proximale (254).

8. Instrument à main (250) selon l'une quelconque des revendications 1 à 7, dans lequel le conduit d'utilité (260) comprend plusieurs tuyaux d'évacuation de fumée/fluide (264).

9. Instrument à main selon l'une quelconque des revendications 1 à 8, dans lequel le conduit d'utilité (260) a un diamètre extérieur d'environ 12,7 mm (0,5 pouces) .

10. Instrument à main (250) selon l'une quelconque des revendications 1 à 9, dans lequel le conduit d'utilité (260) comprend une première section (265A) et une seconde section (265B), la première section et la seconde section étant configurées pour un mouvement de rotation relatif.

11. Instrument à main (250) selon la revendication 10, comprenant en outre un pivot (267) relié entre la première section (265A) et la seconde section (265B) pour faciliter un mouvement de rotation relatif.

12. Instrument à main (250) selon l'une quelconque des revendications 1 à 11, dans lequel la pièce à main (252) est sensiblement creuse entre le réceptacle (268) et l'extrémité proximale (254).

13. Instrument à main (250) selon l'une quelconque des revendications 1 à 12, dans lequel le système de canal (272) comprend un canal longitudinal (274) s'étendant du réceptacle à l'extrémité proximale (254) de la pièce à main (252).

14. Instrument à main (250) selon l'une quelconque des revendications 1 à 13, dans lequel le conduit d'utilité (260) définit un canal d'écoulement intérieur ayant un diamètre généralement uniforme sur la longueur du conduit d'utilité.
